# EUROPÄISCHE PATENTSCHRIFT

(11) **EP 3 023 119 B1**
(45) Veröffentlichungstag und Bekanntmachung des Hinweises auf die Patenterteilung: **26.07.2023**
(21) Anmeldenummer: 15003395.9
(22) Anmeldetag: 10.09.2013
(51) Int. Cl.: A61M 27/00

(54) **EINSTELLBARES HYDROCEPHALUS-VENTIL MIT FEDER**
ADJUSTABLE HYDROCEPHALUS VALVE WITH SPRING
VANNE RÉGLABLE À RESSORT DESTINÉE À TRAITER L'HYDROCÉPHALIE

(30) Priorität: 11.09.2012 DE 102012017886
(43) Veröffentlichungstag der Anmeldung: 25.05.2016
(62) Teilanmeldung aus: 13773598.1
(73) Patentinhaber: Christoph Miethke GmbH & Co. KG, 14469 Potsdam (DE)
(72) Erfinder: Miethke, Christoph, 14469 Potsdam (DE)
(74) Vertreter: Greiche, Albert

(56) Entgegenhaltungen:
- DE-A1-102005 013 720
- US-A- 5 637 083

## Beschreibung

Die Erfindung betrifft ein einstellbares Hydrocephalus-Ventil zum Druckausgleich des Liqor im Schädel eines Hydrocephalus-Patienten.

Hydrocephalus-Patienten haben folgendes medizinisches Problem:
Das Gehirn ist im Schädel von einer besonderen Flüssigkeit, dem Liquor, umgeben. Dieser Liquor wird ständig produziert und im gleichen Maße resorbiert. Bei der Erkrankung des Hydrocephalus, auch Wasserkopf genannt, ist dieses Gleichgewicht gestört, und es wird mehr Flüssigkeit erzeugt als abgebaut wird. Da der Schädelinnenraum ein geschlossenes Gefäß darstellt, kommt es zu einer Volumenvergrößerung. Beim Säugling können die Schädelnähte nicht zusammenwachsen, beim Erwachsenen steigt der Schädelinnendruck. Es gibt also einen Alters- und Kinderhydrocephalus.

Die Behandlung des Hydrocephalus erfolgt ursprünglich durch die bloße Ableitung des Liquors. Dies geschah durch die bloße Schlauchverbindung zwischen dem Schädel und einem großen venösen Blutgefäß oder durch eine entsprechende Verbindung des Schädels über einen Schlauch mit dem Bauchraum. Bald erkannte man jedoch, daß der Druck im Schädel einen bestimmten physiologischen Wert besitzen muß, wenn nicht wieder andere Komplikationen auftreten sollen.

Es sind verschiedene Ventile bekannt, die in die Drainleitung für den Liquor eingebaut werden und mit deren Hilfe der Druck des Liquors eingestellt wird. Derartige Ventile werden im Bereich des Kopfes unter der Haut implantiert. Die Ventile sollen sich bei einem bestimmten kritischen Druck öffnen und den Abfluß von Liquor frei geben. Über eine Leitung - ebenfalls unter der Haut implantiert - wird der Liquor in die obere Hohlvene oder die Bauchhöhle abgeleitet.

Die bekannten Ventile helfen zwar bereits erheblich.

Eine befriedigende Lösung ist mit den bekannten Ventilen jedoch noch nicht erreicht.

Es fehlt an der Einstellung auf den jeweiligen Patienten, d.h. auf den jeweiligen Anwendungsfall.

Es sind schon Ventilentwicklungen bekannt, die eine Einstellung erlauben. Dabei handelt es sich um Ventile, die dem Patienten implantiert werden und vorzugsweise über eine gleichfalls implantierte Schlauchleitung den überschüssigen Liquor aus dem Schädel des Patienten abziehen und vorzugsweise in eine Hohlvene oder in den Bauchraum ableiten. Dabei wird der Ventildruck durch eine Feder bestimmt, wobei die Feder über eine verstellt wird, die ein schwenkbewegliches oder drehbewegliches Teil besitzt, das von außen durch Schwenken oder Drehen des Magneten bewegt wird, so daß die Feder gespannt oder entlastet wird.

Die bekannten Ventile besitzen eine abgeflachte Bauweise. Ziel der flachen Bauweise ist es, beim Implantieren möglichst Beulen am Kopf des Patienten zu verhindern.

Zu den bekannten Ventilen gehört zum Beispiel das Codman Medos Ventil. Dieses Ventil ist ein Kugelventil mit einer federbelasteten Kugel. Die Ventileinstellung erfolgt durch Änderung der Federstellung. Die Feder drückt mit dem einen Ende auf die Ventilkugel. Mit dem anderen Ende stützt sich die Feder auf einem Widerlager ab. Das Widerlager ist bei dieser Bauart durch Drehung höhenverstellbar. Die Höhenverstellbarkeit wird dadurch erreicht, daß das Widerlager schwenkbeweglich angeordnet ist und oben mit einem schräg bzw. treppenartig gestuft verlaufenden Rand versehen ist, auf dem die Feder gleitet. Der Verstellbereich des Widerlagers ist auf eine

Widerlagerschwenkbewegung von maximal 180 Grad beschränkt.

Das führt zu Ungenauigkeiten in der Einstellung. Hinzu kommt, daß eine geringe unbeabsichtigte Schwenkbewegung des Widerlagers bereits zu erheblichen Ventiländerungen führen kann.

*Eine Abwandlung des Codman-Medos-Ventils ist in der* US5637083 *beschrieben. Dieses bekannte Ventil ist gleichfalls ein Hydrocephalusventil mit verstellbarem Ventildruck. Das Ventil besitzt gleichfalls eine federbelastete Ventilkugel. Die Feder ist als Spiralfeder ausgebildet und stützt sich an einem drehbeweglichen Rotor ab. Die Drehung des Rotors wird mit Magneten bewirkt. Dazu sind in dem Rotor Magnete vorgesehen und ist eine Verstellvorrichtung vorgesehen, die gleichfalls Magnete besitzt. Die Drehung des Rotors bewirkt eine Federverstellung. In jeder gewünschten Stellung des Rotors erfolgt eine Verriegelung des Rotors. Dazu greift ein Stift in entsprechende Ausnehmungen des Rotors.*

Zu den bekannten Ventilen gehört auch das Sophysa-Ventil.

Selbst der Hersteller dieses Ventiles äußert Bedenken, wenn der Hydrocephalus-Patient mit Permanentmagneten in Spielzeugen, Kopfhörern, Lautsprechern, elektro-magnetischen Feldern in Berührung kommt, wie sie von elektrischen Motoren, von Rasierern, Haartrocknern, Schaltern usw. ausgehen. Diese Warnung des Herstellers beinhaltet eine Warnung vor den meisten Lebensbereichen der Menschen. Da sich ein Mensch der zeitgemäßen Belastung mit solchen Dingen **nicht** entziehen kann, sind solche Lösungsvorschläge **nicht** praktikabel.

Im einzelnen wird zum Stand der Technik Bezug genommen auf:
DE600024437T2; DE60315924T2; DE29725762T2; DE69808558T2; EP41421557B1; EP688578B1; EP1243826A2; EP1457231B1; EP1604703B1; EP1642613B1; EP255227B1; EP2420284A2; US4443214, US4540400; US4551128; US4673384; US4769002; US5843013; US6840917; US8298168; US2005/0055009; US2012/0197178; US2012/0302937; US2013/0066253; US2013/0085441.

Zwischenzeitlich hat es einige Bemühungen gegeben, die Ventile zu verbessern. Dazu gehört die DE10358145. Dieser Vorschlag hält an der bekannten Bauweise fest und hat sich die Aufgabe gestellt, die Sicherheit solcher Ventile zu erhöhen.

Nach der DE10358145 wird eine höhere Sicherheit auf zwei Wegen erreicht. Der eine Weg beinhaltet eine besondere Verstellung der Feder. Dabei ist ein besonders großer Verstellweg vorgesehen, der in eine Änderung der Federbelastung untersetzt wird. Das heißt, bei vergleichbarem Änderungsbereich der Federbelastung ist ein größerer Verstellweg vorgesehen. In dem Umfang, in dem der Verstellweg größer wird, verringert sich die oben wiedergegebene Gefahr unerwünschter Verstellung.

Vorteilhafterweise erhöht sich zugleich die Genauigkeit der Verstellung mit größer werdendem Verstellweg.

Der andere Weg zur größeren Sicherheit wird von der DE10358145 durch eine magnetbetätigte Verriegelung eröffnet.

Die Möglichkeit zur größeren Gestaltung des Verstellweges ergibt sich durch Änderung der Lage der Feder. Nach der DE10358145 wird die Feder so gelegt, daß die Bewegungsebene der Feder bei deren Verstellung parallel zur Ebene liegt, in der die Schwenkbewegung oder Drehbewegung des Schwenkteiles/Rotors stattfindet. Dabei ist die Parallelität auch dann gegeben wenn die Ebenen zusammenfallen.

Durch die besondere Anordnung der Feder kann die Feder sich in der Richtung bewegen, in der sich das Ventilgehäuse am weitesten ausdehnt. Das ist die Richtung der Flachseite.

Vorzugsweise findet als Feder ein Federstab Anwendung, der schwenkbeweglich angeordnet ist und dessen eines Ende länger als das andere Ende ist. Das kürzere Ende steht mit der einer Ventilkugel oder Ventilklappe des Ventils in Wirkverbindung, das längere Ende ermöglicht den größeren Verstellweg und wirkt mit der beschriebenen Verstellmechanik zusammen. Dabei ist eine gleitende, an sich bekannte Wirkverbindung zwischen der Feder und dem drehbeweglichen oder schwenkbeweglichen Teil vorgesehen. Das heißt, die Feder gleitet auf einer Fläche des drehbeweglichen oder schwenkbeweglichen Teils der Verstellmechanik.

Günstig ist die Verwendung eines Federstabes, der als doppelarmiger Hebelarm ausgebildet ist. Der doppelarmige Hebelarm ist gelenkig gelagert.

Die Wirkverbindung mit der Ventilkugel bzw. der Ventilklappe wird dadurch gebildet, daß das kürze Ende gleitend gegen die Ventilkugel bzw. die Ventilklappe drückt.

Die Wirkverbindung mit der Verstellmechnik wird dadurch gebildet, daß an dem drehbeweglichen oder schwenkbeweglichen Teil/Rotor eine Gleitfläche für den kürzeren Hebelarm vorgesehen ist, die als Kurvenbahn ausgebildet ist, an der der Federstab gleitend anliegt.

Die Kurvenbahn läuft nach der DE10358145in Umfangsrichtung und in radialer Richtung an dem schwenkbeweglichen oder drehbeweglichen Teil. Der Umfangswinkel an dem schwenkbeweglichen bzw. drehbeweglichen Teil umfaßt insbesondere mindestens 300 Grad.

Die den Ventildruck bestimmende Feder kann an der Kurvenbahn in einer Schwenkrichtung/Drehrichtung oder in beiden Schwenkrichtungen/Drehrichtung gleiten. Die Bewegungsrichtung ergibt sich aus der Drehrichtung bzw. Schwenkrichtung des drehbeweglichen bzw. schwenkbeweglichen Schenkteiles/Rotors.

Wahlweise kann das drehbewegliche Schwenkteil/Rotor auch in der gleichen Drehrichtung weiterbewegt werden und gleichwohl wieder an den Verstellanfang kommen. Das wird dadurch erreicht, daß zwischen dem Anfang der Kurvenbahn und dem Ende der Kurvenbahn an dem drehbeweglichen oder schwenkbeweglichen Teil eine Verbindung vorgesehen ist.

Das schwenkbewegliche bzw. drehbewegliche Schwenkteil/Rotor sitzt auf einer Achse/Zapfen/Bolzen, der mit dem flexiblen Deckel oder Boden des Gehäuses einstückig ist. Mit der Achse/Zapfen/Bolzen ist das Schenkteil/Rotor im Gehäuse des Ventiles drehbeweglich bzw. schwenkbeweglich gelagert.

Die auf dem schwenkbeweglichen Schenkteil/Rotor gleitende, den Ventildruck bestimmende Feder hat vorzugsweise eine Winkelform. Die beiden Hebelarme des doppelarmigen Hebelarmes stehen in einem Winkel zueinander, der vorzugsweise kleiner als 180 Grad ist, auch kleiner als 90 Grad sein kann.

Der Querschnitt der den Ventildruck bestimmenden Feder kann beliebig sein. Günstig sind runde und rechteckige Formen. Besonders günstig ist eine Feder mit blattförmigem oder drahtförmigem Querschnitt.

Zur schwenkbeweglichen bzw. drehbeweglichen Lagerung der den Ventildruck bestimmenden Feder eignet sich zum Beispiel ein Stift/Achse/Bolzen, dessen Enden in entsprechende Ausnehmungen im Ventilgehäuse bzw. im Ventildeckel oder Ventilboden greifen. Die Enden des Stiftes können auch spitz ausgebildet sein, so daß der Stift in den Ausnehmungen auf den Spitzen dreht. Diese Vorgehensweise ist technisch und wirtschaftlich günstig.

Zur Befestigung des Stiftes an der Feder eignet sich eine Schweiß- oder Lötverbindung, auch andere Verbindungen.

Für die Funktion der den Ventildruck bestimmenden Feder ist günstig, wenn der lange Hebelarm an dem schwenkbeweglichen Teil bzw. drehbeweglichen Teil(Schwenkteil/Rotor) der Verstellmechanik geführt ist. Dazu kann dieses Schwenkteil/Rotor zugleich an mindestens einer Seite die den Ventildruck bestimmenden Feder führen. An der anderen Seite kann die Führung zum Beispiel durch eine Scheibe oder durch eine Membran oder durch einen Gehäusedeckel oder Gehäuseboden gebildet werden.

Ventilkugelseitig ist es günstig, wenn eine großflächige Berührung zwischen der den Ventildruck bestimmenden Feder und der Ventilkugel stattfindet. Soweit die den Ventildruck bestimmende Feder diese großflächige Berührung nicht hergibt, kann an dem betreffenden Federende ein Blech befestigt werden. Das Blech ist wahlweise angeschweißt oder angelötet oder in sonstiger Weise befestigt.

Das Schwenkteil/Rotor wird üblicherweise mit Magneten bewegt, die in das Schwenkteil/Rotor eingelassen sind. Das Schwenkteil/Rotor mit Magneten und die den Federdruck bestimmende Feder werden im Folgenden als Verstellmechanik des implantierten Ventils bezeichnet. Zur Verstellung des Ventildruckes wird außen auf der Haut des Patienten ein Verstelleinrichtung aufgesetzt, welche auch mit Magneten versehen ist. Diese Magnete der außen aufgesetzten Verstelleinrichtung wirken auf die Magnete des Schwenkteiles/Rotors in dem implantierten Ventil, so daß eine Drehung/Verschwenken der außen liegenden Verstelleinrichtung eine Drehung/Verschwenken des in dem implantierten Ventil angeordneten Schwenkteiles/Rotors bewirkt. Dem folgt ein Verschwenken der den Ventildruck bestimmenden Feder, verbunden mit einer Änderung des Ventildruckes.

Seit den 90iger Jahren ist bekannt, daß die Verstellmechanik auch unbeabsichtigt verstellt werden kann, wenn der Patient in den Wirkungsbereich starker magnetischer Felder gelangen kann.

Das hat zu dem Wunsch geführt, die Verstellmechanik in jeweiligen Stellung zu verriegeln. Es sind zur Verriegelung verschiedene Vorschläge bekannt. Bekannte Vorschläge nutzen für die Verriegelung wiederum Magnete.

Andere Vorschläge benutzen für die Verriegelung Federkräfte und Reibungskräfte.

Durchgesetzt haben sich bisher zwei Vorschläge.

Bei dem einen Vorschlag drückt ein Gehäuse im Verriegelungszustand mit Nocken reibungsschlüssig auf den schwenkbeweglichen Rotor/Schwenkteil, der die Schrägfläche bzw. treppenförmig gestufte Fläche trägt. An der die oben beschriebene Feder gleitet. Zur Entriegelung muß das Gehäuse so gewölbt werden, daß die Nocken von dem Rotor/Schwenkteil abheben. Nach dem Abheben der Nocken kann der Rotor/Schwenkteil mit einer außen auf der Haut des Patienten liegenden Verstelleinrichtung verschwenkt werden. Diese Verstelleinrichtung besitzt Magnete, die mit anderen Magneten zusammen wirken, die im Rotor/Schwenkteil angeordnet sind.

Bei dem anderen Vorschlag wird die Federkraft eines gewölbten Gehäusedeckels zur Verriegelung genutzt. Der Deckel zieht den Rotor/Schwenkteil mit seiner aus der Wölbung entstehenden Kraft gegen sich, so daß im Verriegelungszustand Reibungsschluß des Rotors/Schwenkteiles mit dem Gehäusedeckel entsteht. Dabei sitzt der Rotor/Schwenkteil drehbeweglich/schwenkbeweglich auf einer an dem Gehäusedeckel vorgesehenen Achse/Bolzen.

Für die Entriegelung wird der Gehäusedeckel verformt, so daß der Rotor/Schwenkteil von dem Gehäusedeckel abhebt, kein Reibungsschluß mehr zu dem Deckel besteht und mit Hilfe einer außen auf der Haut des Patienten aufgesetzten Verstelleinrichtung verschwenkt werden kann. Die Verstelleinrichtung besitzt Magnete, die in das Schwenkteil/Rotor eingelassen sind.

Mit dem drehbeweglichen Schwenkteil/Rotor wird je nach Drehrichtung/Schwenkrichtung die an dem Schwenkteil/Rotor gleitende, den Ventildruck bestimmende Feder gespannt oder entspannt. Dabei gleitet die den Ventildruck bestimmende Feder auf einer Kurvenbahn des Schwenkteiles/Rotors. Zugleich drückt die den Ventildruck bestimmende Feder drückt auf die Ventilkugel des Ventils, so daß es zu einer gewünschten Veränderung des Schließdruckes der Ventilkugel bzw. des Ventiles kommt.

Die Ventilkugel wirkt mit einer kegeligen Öffnung im Gehäuse des Ventiles zusammen. Diese Öffnung ist bei der DE10358145 eintrittsseitig am Ventil angeordnet.

Als Magnete finden vorzugsweise kleine Bauformen, zum Beispiel Stiftmagnete, Verwendung. Die kleinen Magnete tragen auch zu geringen Ventilabmessungen bei.

Die außen auf der Haut des Patienten vorgesehene Verstelleinrichtung für das Ventil kann gleichfalls mit extrem kleinen Abmessungen gestaltet werden. Nach der DE10358145 wird das zur Reduzierung des Durchmessers der Verstelleinrichtung und zu einer besonderen Formgebung der Verstelleinrichtung genutzt, nämlich zur Gestaltung der Verstelleinrichtung in Stiftform, ähnlich einem Kugelschreiber. Das erlaubt die Handhabung der Verstelleinrichtung wie die Handhabung eines Stiftes oder Kugelschreibers, z. B durch Tragen in einer Brusttasche. Zugleich wird die Mechanik eines Kugelschreibers genutzt, um die am Kopf der Verstelleinrichtung vorgesehenen Magnete in Längsrichtung des Stiftes vor (bei aufgesetztem Stift gegen den Kopf des Patienten bzw. gegen das Ventil) oder zurück zu bewegen. Bei vertikaler Lage des Stiftes ist das ein Heben und Senken.

Wahlweise besitzt die stiftförmige, außen auf der Haut des Patienten aufgesetzt Verstelleinrichtung am vorderen Ende eine Kappe, mit dem die Verstelleinrichtung aufgesetzt wird. Bei lockerem Aufsetzen der Verstelleinrichtung sollen die die Magnete selbsttätig eine Zentrierung der Verstelleinrichtung über dem Rotor/Schwenkteil des Ventil bewirken.

Nach der Zentrierung der außen am Patienten angeordneten Verstelleinrichtung auf dem implantierten Ventil ist eine elastische Verformung des implantiertenVentils zum Zwecke der Entriegelung des Schwenkteiles/Rotors vorgesehen. Anschließend erfolgt die oben beschriebene Verstellung.

Die in der DE10358145 beschriebene Technik hat sich *bewährt. Eine andere bewährte Form ist zum Beispiel in der* DE10 2004 013 720 *beschrieben. Diese Druckschrift zeigt ein einstellbares Hydrocephalus- Ventil zum Druckausgleich des Liquor im Schädel eines Hydrocephalus-Patienten, Das Ventil ist dem Patienten implantierbar und vorzugsweise mit einer gleichfalls implantierbaren Schlauchleitung, über die überschüssiger Liquor aus dem Schädel des Patienten abziehbar und in die obere Hohlvene oder in den Bauchraum drainierbar. Der Ventildruck wird durch eine Feder bestimmt, wobei die Feder über eine Verstellmechanik verstellt wird, so daß die Feder gespannt oder entlastet wird, Zu der Verstellmechanik des Ventils gehört ein schwenkbewegliches oder drehbewegliches und im Ventil angeordnetes Schwenkteil*/*Rotor, der mit Magneten versehen ist und von außen durch Schwenken oder Drehen einer Verstelleinrichtung bewegbar ist, die ihrerseits mit Magneten versehen ist und von außen durch Schwenken oder Drehen einer Verstelleinrichtung bewegbar ist, so daß zwischen zwei Verstellvorgängen eine Arretierung des Schwenkteiles*/*Rotors erfolgen kann. Dabei erfolgt die Verriegelung durch Reibungsschluss zwischen dem Schwenkteil*/*Rotor und dem Gehäuse und wird der drehbewegliche oder schwenkbewegliche Schwenkteil*/*Rotor zur Aufhebung des Reibungsschlusses unter Eindrückung des Ventildeckels in axialer Richtung bewegt. Die Entriegelungsbewegung erfolgt gegen eine Federkraft, die bei der Verriegelung den Reibungsschluss bewirkt, wobei der Ventildeckel mit einer eindrückbaren Ausbeulung versehen ist*.

Ein weiteres Ventil ist aus der DE102005013720 bekannt. Die Merkmale des Oberbegriffs des Anspruchs 1, können dieser Schrift entnommen werden.

Gleichwohl hat sich die Erfindung die Aufgabe gestellt, die Technik zu verbessern. Dabei ist die Erfindung von der Erkenntnis ausgegangen, daß das Ventil und seine Bedienung noch verbesserungsfähig sind.

Nach der Erfindung wird das mit den Merkmalen des Hauptanspruches und in bevorzugter Ausführung mit den Merkmalen der Unteransprüche erreicht.

Dabei ist hervorzuheben, daß das Ventil bei der Entriegelung und/oder bei der Verriegelung ein Signal gibt. *Keines der anderen oben beschdriebenenen Ventile zeigt eine Signalgebung. Das gilt auch für die aus der* US 5637083 *und für das aus der* DE102005013720 *bekannte Ventil.*

Es können auch Signale zur Kontrolle der Lage des drehbeweglichen oder schwenkbeweglichen Schwenkteiles/Rotors genutzt werden. Die Signale können akustisch und/oder optisch und/ oder elektronisch oder fühlbar sein. Mit den Signalen wird eine hohe Sicherheit für eine richtige Platzierung der Verstelleinrichtung geschaffen. Nur durch richtige Platzierung kann geklärt werden, welche Stellung die Ventile aktuell haben und anschließend die richtige Änderung der Stellung vorgenommen werden.

Wahlweise kommen elektronische Signalgeber zum Einsatz.

Bei elektronischen Signalgebern kann das Signal nach Wahl in optischer und/oder akustischer Form oder als gefühltes Signal erzeugt werden. Das schließt eine Fernübertragung des im subkutan angeordneten Ventil erzeugten Signals zu einem außen liegenden Signalempfänger und eine Signalumwandlung in dem außen liegenden Signalempfänger ein.

Als elektronische Signalgeber können passive Transponder im Ventil von besonderem Vorteil sein. Die passiven Transponder sprechen auf Signale an, die von außen eingegeben werden, und geben dann von sich aus ein Signal nach außen.

Vorzugsweise wird das Signal mechanisch erzeugt. Noch weiter bevorzugt werden Knackgeräusche erzeugt. Erfindungsgemäß entstehen die Knackgeräusche durch Verwendung von einem durch ein Federblatt gebildetes Ventildeckel, das eine Beule aufweist, die durch Eindrücken schlagartig in eine gegenüber liegende Beule bringbar ist, so daß ein Geräusch entsteht.

Nach der Erfindung wird das elastische Material zur Verformung gebracht und danach schlagartig frei gegeben, so daß sich je nach Art der Verformung das Material schlagartig weiterverformen oder schlagartig zurückverformen kann. Als Material kommt jedes biokompatible elastische Material in Betracht. Dazu gehören Metalle wie auch Kunststoffe, auch Verbundstoffe. Wahlweise bestehen die Federbleche aus dem gleichen Material wie die den Ventildruck bestimmende Feder. Vorzugsweise findet Titan(Titanlegierungen) für die Federbleche Anwendung.

Da es keine immer währende absolute Elastizität gibt, sollen nur solche Materialien Anwendung finden, die während der Lebensdauer eines Ventils durch die erfindungsgemäße Verformung höchstens 50% bleibende Verformung, vorzugsweise höchstens 30% bleibende Verformung und weiter bevorzugt höchstens 10% bleibende Verformung und höchst bevorzugt höchstens 5% bleibende Verformung erfahren, wobei die %-Angaben sich auf das Maß beziehen, um das sich die Rückformung nach der letzten geräuschbildenden Verformung des Federstahls während der Lebensdauer des Ventils gegenüber der Rückverformung nach der ersten geräuschbildenden Verformung reduziert.

Vorzugsweise wird blattförmiges Material aus Titan (Titanlegierungen) bzw. aus dem gleichen Metall gewählt, aus dem auch das Gehäuse des Hydrocephalusventils besteht. Dieses Material ist biokompatibel. Für die Federbleche aus Titan bzw. aus dem gleichen Metall wie die das Ventilgehäuse gilt hinsichtlich der Elastizität/bleibenden Verformung das gleiche wie für Federbleche aus Stahl.

Erfindungsgemäß finden blattförmige Federn/Federbleche für die geräuschbildende Verformung Anwendung.

Für die Verformung der Blattfeder muß ein entsprechender Raum in dem Ventil zur Verfügung stehen. Dabei ist es von Vorteil, wenn das Federblatt ein Bestandteil des Ventilgehäuses ist. Erfindungsgemäß bildet das Federblatt zugleich den Ventildeckel. Dabei kann das den Ventildeckel bildende Federblatt auch als Membran bezeichnet werden. Der Ventildeckel ist nach der Erfindung nicht der immer oben liegende Ventilteil, sondern immer der Teil des implantierten Ventils, das so im Patienten implantiert ist, daß sein Ventildeckel mit einer außen auf der Haut des Patienten aufgesetzten Verstelleinrichtung korrespondiert. Dabei ist das Ventil nicht an eine Implantation unter der Kopfhaut des Patienten gebunden. Auch wenn das die regelmäßige Implantationsstelle ist, kann das Ventil auch an einer anderen Körperstelle implantiert werden.

Vorzugsweise ist ein kreisrundes Federblatt mit Beulstruktur als Ausgangsform vorgesehen. Noch weiter bevorzugt ist die Beulstruktur, welche die Ausgangsform bildet, die durch Kaltverformung erzeugt geworden ist. Kreisrunde Federblätter, die eingedrückt werden, sind an Hydrocephalusventilen bekannt. Die bekannten Federblätter verursachen jedoch kein Geräusch.

Für die Geräuschbildung ist ein Spannungszustand im Federblatt maßgebend, Mit der Beule in dem Federblatt, ergibt sich im Unterschied zur Eindrückung eines ebenen Federblattes eine besondere Kraft-Weg-Kennlinie. Beim Eindrücken steigt die Kennlinie zunächst an. Dabei wächst der Widerstand gegen das Eindrücken aufgrund der einzudrückenden Beulstruktur stärker an als bei der Verformung eines ebenen Federblattes. Beim Überschreiten einer bestimmten Kraft und Erreichen eines bestimmten Verformungszustandes fällt die Linie steil und sprunghaft ab. Die Kraft-Weg-Kennlinie knickt ab und dadurch entsteht in der Mechanik bekannter Druchschlag-Effekt. Das heißt, das Federblatt schlägt auf der anderen Seite durch und gibt ein akustisches und haptisches Geräusch, das als Knacken bezeichnet wird. Dieser Effekt wird bei Spielzeugen benutzt, den sogenannten Knack-Fröschen. Das Durchschlagen ist auch fühlbar. Dieser Effekt wird auch beim Abrichten/Dressieren von Tieren benutzt. Dort werden die Geräte als Klicker bezeichnet.

In der Technik ist diese Geräuschbildung auch bei Schnappschaltern, Klappschaltern und Kippschaltern bekannt. Dabei ist nicht die Geräuschbildung sondern die schlagartige Berührung der Schaltflächen angestrebt, um Stromspitzen aus dem Schaltvorgang zu vermeiden.

Mit dem Durchschlagen des Federblattes entsteht auf der gegenüberliegenden Seite des Federblattes eine Beule.

Nach Beenden der Belastung gibt es zwei Möglichkeiten:
a)In der einen Variante ergibt sich ein stabiler Zustand. Die Beule bleibt nach dem Durchschlagen auf der Seite, auf der sie entstanden ist. Das heißt, die Blattfeder hat zwei stabile Beul-Zustände, die Beule vor der Verformung und die Beule nach der Verformung. Um wieder nach einer Verformung zu einer Rückverformung zu kommen, ist vorzugsweise eine Mechanik vorgesehen, mit der die Beule wieder eingedrückt werden kann. Mit der Mechanik kann ein Gegendruck gegen die entstandene Beule erzeugt werden, der die Rückverformung in Gang setzt. Wahlweise können dazu die Achse, auf der das Schwenkteil/Rotor sitzt und eine auf die Achse wirkende Feder genutzt werden. Zusätzlich kann das Schwenkteil/Rotor auch für die Rückverformung genutzt werden. Das gilt besonders für den Fall, daß das Schwenkteil/Rotor zur Verriegelung reibungsschlüssig mit dem Ventildeckel in Berührung gebracht wird und wenn das Verstellteil/Rotor mit der zugehörigen Achse von einer Feder in Richtung des Ventildeckels gedrückt wird, die unabhängig von dem Ventildeckel ist. Dann wird diese Feder zwar beim Entriegeln zusammengedrückt. Beim Verriegeln wird der Druck jedoch wieder von dieser Feder genommen, weil die außen auf der Haut des Patienten stehende Verstelleinrichtung entfernt wird. Dann kann die Feder nicht nur den Verriegelungsvorgang bewirken, sondern zugleich auch die durch den ersten Verformungsvorgang entstandene Beule zur Rückverformung anstoßen. Der Anstoß muß lange nicht mit der Kraft ausgeübt werden, der für den ersten Verformungsvorgang erforderlich ist.

In dem Federblatt ist durch den ersten Verformungsvorgang ein Spannungszustand entstanden, der die Rückverformung wesentlich unterstützt. Dabei ist von Vorteil, wenn das Federblatt als Wellmembran ausgebildet ist wie das in der PCT/EP2011/003903 beschrieben ist. Noch günstigere Spannungszustände ergeben sich, wenn das Federblatt kreisförmig ausgebildet und in eine bleibende Form tiefgezogen worden ist, die eine gestufte Beule bildet.

Die erfindungsgemäße Beulstruktur hat den weiteren Vorteil zusätzlicher Federeigenschaft, größerer Stabilität und größerer Festigkeit, so daß auch eine etwas exzentrische Krafteinleitung beim Entriegeln und Verstellen des Ventils unschädlich ist.

Außerdem wird das Geräusch eines durchschlagenden erfindungsgemäß gewellten oder gestuft tiefgezogenen Federblattes angenehmer als das Geräusch eines durchschlagenden ungestuften/ungewellten Federblattes empfunden.

Außerdem entfällt der Kraftaufwand bei der Rückvorverformung, welcher bei der ersten Verformung erforderlich war, um die den Reibungsschluß bei der Verriegelung verursachende Feder zusammen zu drücken.

Von Vorteil für die Rückverformung ist auch, wenn der Gehäusedeckel zweischalig ist, wobei die äußere Schale das oben erläuterte Federblatt bildet und die zweite, innen liegende Schale die Verformung der ersten, außen liegenden Schale/Federblattes begrenzt. Diese Begrenzung kann auch als Widerlager bezeichnet werden, weil sich die erste Schale bei der ersten Verformung an die zweite, innen liegende Schale anlegt.

Vorzugsweise wird die Verformung der außen liegenden Schale unmittelbar nach der Geräuschbildung mit Hilfe der zweiten, innen liegenden Schale beendet. Auch dadurch reduziert sich die für das Anstoßen der Rückverformung erforderliche Federkraft.

Vorteilhafterweise endet die Rückverformung gleichfalls unter Geräuschbildung. Das gibt zusätzliche Sicherheit zur richtigen Ventilverstellung.
b)in der zweiten Variante wird bei sonst gleicher Ausbildung wie bei der Variante a) ein Federblatt mit einer kalt eingeformten, bleibenden Beulstruktur verwendet wird. Das Federblatt der Variante b) unterscheidet sich von dem Federblatt der Variante a) dadurch, daß das Federblatt keines Anstoßes bedarf, um sich nach Beendigung der Verformung zurückzubilden. Das wird darauf zurückgeführt, daß die durch die Kaltverformung erzeugten Spannung zusammen mit den Spannungen aus dem Eindrücken der Beulstruktur groß genug sind, um die selbstätige Rückverformung zu bewirken, gleichwohl aber noch den Effekt des geräuschbildenden Durchschlagens zeigen.

Dieses Federblatt hat den Vorteil gegenüber dem Federblatt nach Variante a), daß es die Aufgabe erleichtert und für eventuelle plastische Verformung durch Fehlbedienung eine Reserve bzw. Sicherheit bietet, daß das Federblatt sich in der gewünschten Form zurückbildet. Diese Feder wurde mit einer Belastung von 10kg getestet. Mehr kann ein Arzt bei der Verstellung des Ventildruckes nicht aus Versehen von Hand verursachen. Im Ergebnis hat sich die erste, äußere Schale des Gehäusedeckels, welche das Federblatt bildet zwar plastisch verformt. Die Kraft-Weg-Kennlinie hat sich so verändert, daß die erste Schale des Gehäusedeckels bei geringerer Kraft als vorher ein Geräusch erzeugt hat, aber immer noch die gewünschte Entriegelung bewirkt hat und sich nach der Entlastung wieder so weit zurückgebildet hat, daß die Verriegelung stattfinden und nachfolgende Entriegelungs- und Verriegelungsvorgänge nicht gestört wurden.

Der erfindungsgemäße zweischalige Ventildeckel kann auch ein mit dem Ventilgehäuse einstückiges oder ein an dem Ventilgehäuse angeschweißtes Teil sein. Vorzugsweise wird der zweischalige Ventildeckel durch einen topfförmigen Teil des Ventilgehäuses gebildet bzw. bildet der Ventildeckel mit einem Teil des Ventilgehäuses eine Topfform. Noch weiter bevorzugt wird die Topfform aus mehreren Teilen zusammen gesetzt. Dazu gehören ein ringförmiger Teil des Ventilgehäuses sowie die beiden Schalen des Ventildeckels. Die beiden Schalen können mit dem ringförmigen Gehäuseteil verschweißt werden oder auch in anderer Weise verbunden werden.

Vor dem Verschweißen werden die beiden Schalen in die gewünschte Form gebracht. Das kann durch Pressen/Tiefziehen eines ebenen Materials erfolgen. Für den gewünschten Spannungszustand ist ein Pressen/Tiefziehen in kaltem Zustand von Vorteil.

Die Verformung des Ausgangsmaterials für die Schalen des Ventildeckels kann in einem oder mehreren Schritten/Stufen stattfinden. Bei mehrstufiger Herstellung wird vorzugsweise zunächst eine konzentrische Wellenstruktur in der Ebene bzw. mit geringen Höhenunterschieden erzeugt, bevor in eine zweiten Schritt eine abschließende dreidimensionale Verformung stattfindet.

Für die Verformung ist vorzugsweise eine Presse vorgesehen. Es gibt mechanisch betätigte Pressen, hydraulische Pressen und mit Druckluft betriebene Pressen. Hier ist die erforderliche Presskraft gering, weil die Federblätter klein und dünn sind. Deshalb können hier auch handbetätigte Pressen, insbesondere Kniehebelpressen zur Anwendung kommen.

Die Preßwerkzeuge bestehen aus Matrize und Patrize. Es sind für jede Verformungsstufe separate Matrizen und Patrizen vorgesehen, also im Falle eines zweistufigen Verformungsvorganges für den ersten Verformungsschritt ein Paar aus Matrize und Patrize und für den zweiten Verformungsschritt ein anderes Paar aus Matrize und Patrize.

Bei geformter äußerer Schale ist die das Widerlager bildende innere Schale der der äußeren Schale vorzugsweise angepaßt. Die dazu erforderliche Formgebung muß die Form der äußeren Schale nicht identisch widergeben. Es ist ausreichend und mit geringerem Aufwand verbunden, wenn die innere Schale mit ebenen Stufen versehen ist, auf denen die äußere Schale mit ihren Auswölbungen zur Anlage kommt. Vorteilhafterweise kann eine innere Schale als Widerlager für verschiedene äußere Schalen dienen.

Die Zweischaligkeit am Ventilgehäuse ist auch unabhängig von der Geräuschbildung, desgleichen unabhängig davon von Vorteil, ob die Achse, auf der das Schwenkteil sitzt mit dem Gehäusedeckel verbunden ist oder ein separates Bauteil bildet.

Die erfindungsgemäßen Ventile besitzen vorzugsweise einen Durchmesser von 8 bis 20mm, noch weiter bevorzugt bis 15mm.

Wahlweise orientiert sich die Größe des Federplattes an der Größe des Ventils. Es kann aber auch ein Federblatt einheitlicher Größe für die Ventile Verwendung finden. Dann wird das Federblatt bei größeren Ventilen in den Ventildeckel eingelassen.

Die Dicke des Federblattes wird vorzugsweise nach dessen Durchmesser bzw. nach dessen Größe gewählt. Federblätter für ein Ventil mit einem Durchmesser von 17mm, können eine Dicke von zum Beispiel kleiner/gleich 0,2mm, auch kleiner/gleich 0,16mm aufweisen. Bei kleiner werdendem Ventildurchmesser und entsprechend kleinerem Federblatt kann die Dicke kleiner/gleich 0,15mm betragen.

Ob und welche Verformung das Federblatt erhalten soll, hängt von dessen Funktion ab. Soweit das Federblatt nur in zweischaliger Ausbildung des Ventildeckels zur Entriegelung der Verstellmechanik bestimmt ist, reicht schon ein ganz geringer Hub des Federblattes, um den Reibungsschluß zwischen dem Schwenkteil/Rotor und dem Ventildeckel aufzuheben. Bei guter Ventilqualität kann der notwendige Hub 0,1mm oder weniger betragen. Ein größerer Hub als 0,3mm ist regelmäßig nicht erforderlich. Für einen kleinen Hub muß das Federblatt nicht ausgebeult werden.

Mit Hub ist bei der erfindungsgemäßen Beulung der größte Abstand zwischen den beiden Schalen einer zweischaligen Ventildeckelausbildung bezeichnet. An der Stelle findet der größte Verformungsweg beim Eindrücken des Ventildeckels statt. Bei einschaliger Ventildeckelausbildung ist mit Hub auch der größte Verformungsweg des Ventildeckels bezeichnet.

Für eine erfindungsgemäße Anwendung der zweischaligen Bauweise zur Erzeugung eines Knackgeräusches ist regelmäßig ein größerer Hub erforderlich, zum Beispiel für einen Ventildurchmesser von 17mm ein Hub von mindestens 0,4 mm. Je nach Durchmesser des Ventils und Größe des Federblattes und je nach gewünschter Geräuschbildung bzw. je nach dem Umfang des Durchschlagens der Beulstruktur ist ein größerer oder kleiner Hub erforderlich. Bei der Geräuschbildung sind die Wünsche des Patienten und die Wahrnehmungsfähigkeit des behandelnden Arztes zu berücksichtigen. Die Geräusche werden umso geringer, je geringer der Hub ist. Außerdem kann eine wellenförmige Beulstruktur auf die Geräuschbildung dämpfend wirken. Vorzugsweise beträgt der Hub der äußeren Schale 0,3 bis 2mm.

Der gewünschte Hub bestimmt das Maß der bleibenden Beulstruktur.

Es gibt verschiedene Wege zur Erzeugung der bleibenden Beulstruktur. Bei der bevorzugten zweistufigen Kaltverformung mit dem Prägen der Wellenform/Wölbungsform in der ersten Verformungsstufe und der abschließenden dreidimensionalen Verformung wird das Federblatt über die gewünschte bleibende Form hinaus verformt, um der Elastizität der Federblätter Rechnung zu tragen. Je dünner das für die Federblätter verwendete Material ist, desto schonender sollen die Federblätter verformt werden. Das schließt vorzugsweise ein, daß die Federblätter in der zweiten Verformungsstufe nicht mehr gepresst sondern lediglich tiefgezogen werden. Bei dem Tiefziehen werden die Federblätter am Rand eingespannt und mit einem entsprechenden Formteil ausgebeult, so daß das Material im Bereich der bleibenden Verformung extrem gut fließen kann.

Für eine Anwendung des zweischaligen Ventildeckels mit Beulstruktur ohne Erzeugung eines Geräusches können alle Beschränkungen entfallen, die mit der Geräuscherzeugung verbunden sind. Das betrifft insbesondere die Beschränkung des Hubs aus Gründen der Geräuschdämpfung.

Gleichwohl geht von einem solchen Ventil noch ein Signal aus, weil das Nachgeben der äußeren Schale des Ventildeckels für den behandelnden Arzt fühlbar ist.

Vorteilhafterweise können die Ventildeckel auch in einschaliger Bauweise mit der oben erläuterten Beulstruktur versehen werden. Dann ist der Ventildeckel jedoch mit einer solchen Dicke versehen, daß er allein (ohne ein Widerlager) den Belastungen aus dem Eindrücken Stand hält. Die erfindungsgemäße Beulstruktur entfaltet auch in dieser Anwendung Vorteile.

Von Vorteil ist auch, wenn das Schwenkteil/Rotor anders als nach der DE10358145 auf einer Achse/Zapfen/Bolzen sitzt, der in Bezug auf den zur Verriegelung und Entriegelung des Schwenkteils/Rotors flexiblen Ventildeckeln ein separates Bauteil bildet, das in dem Ventilgehäuse in Längsrichtung verschiebbar gehalten ist. Die zusätzliche verschiebbare Lagerung in Längsrichtung erscheint bei anfänglicher Betrachtung aufwendiger als die mit einem Ventildeckel einstückige Bauweise. Bei näherer Betrachtung hat die separate Ausbildung insbesondere in Kombination mit einem sich zur Geräuschbildung schlagartig ausbeulenden Ventileckel jedoch bauliche Vorteile. Außerdem gewinnt das Ventil mit der verschiebbaren Lagerung noch Genauigkeitsvorteile und Betriebssicherheit.

Ferner eröffnet sich mit der Verwendung einer von dem Ventildeckel separaten Achse/Zapfen/Bolzen die Möglichkeit eines von der Gehäuseverformung unabhängig erzeugten Verriegelungsdruckes.

Nach der Erfindung wird der Verriegelungsdruck mit einer zusätzlich vorgesehenen Feder erzeugt. Für diese zusätzliche Feder kommen verschiedene Federn in Betracht. Vorzugsweise handelt es sich um eine Spiralfeder, die auf der Achse/Zapfen/Bolzen sitzt, mit der das Schwenkteil/Rotor geführt wird. Die Feder hat den Vorteil einer einfacheren Auslegung des Verriegelungsdruckes. Außerdem kann der Verriegelungsdruck durch Längenänderung der Feder bzw. durch Auswechselung der Feder leicht verändert werden.

Für die Verriegelung und Entriegelung ist ein geringer Hub der Achse/Zapfen/Bolzen erforderlich. Vorzugsweise ist der Hub begrenzt.

Zur Begrenzung kann die Achse/Zapfen/Bolzen mit einem Ring oder Bund versehen sein. Der Ring ist zum Beispiel aufgepreßt. Der Bund ist mit der Achse/Zapfen/Bolzen vorzugsweise einstückig.

Der Ring oder Bund wirkt mit einem Anschlag im Gehäuse zusammen, Vorzugsweise wird die Bewegung der Achse/Zapfen/Bolzen, auf dem das Schwenkteil/Rotor sitzt, durch den Ventilboden begrenzt, der dem Ventildeckel am Ventil gegenüber liegt. Um zugleich die Reibungskräfte zwischen dem Ventilboden und der Achse/Zapfen/Bolzen zu reduzieren kann dieser ventilbodenseitig mit einer Spitze versehen sein.

Vorzugsweise ist das Schwenkteil/Rotor im Übrigen mit Markierungen versehen, welche durch Röntgen eine Information über die Lage des Schwenkteils/Rotors geben. Die Informationen können aber auch durch Signale erzeugt werden, die wie die Signale zur Verriegelung/Entriegelung erzeugt werden.

Durch Röntgen oder durch weitere Signale kann im Zweifel geklärt werden, ob das Ventil richtig oder falsch implantiert worden ist. Dazu eignen sich Markierungen, die sich beim Röntgen von der einen Seite anders darstellen als von der anderen Seite.

Als Markierungen eignen sich auch Bohrungen im Schwenkteil/Rotor, wenn die Bohrungen in unverwechselbarer Weise angebracht sind und wenn die Bohrungen mit bestimmten anderen Gehäusebesonderheiten in unverwechselbarer Weise in Beziehung stehen. Solche Gehäusebesonderheit kann ein Anschlag für das Schwenkteil/Rotor oder eine Besonderheit am Ventileintritt oder am Ventilaustritt sein. Die Markierungen können unabhängig von der erfindungsgemäßen Geräuscherzeugung und auch unabhängig von der oben beschriebenen erfindungsgemäßen Ausbildung/Lagerung des Schwenkteiles/Rotors auch bei anderen Bauarten von Hydrocephalusventilen Anwendung finden.

Bei dem erfindungsgemäßen Ventil ist wahlweise auch eine Begrenzung des Schwenkwinkels vorgesehen. Zur Begrenzung der Schwenkbewegung können Bolzen oder Zapfen oder sonstige Anschläge in dem Gehäuse vorgesehen sein.

Wahlweise sind gegenüber der DE10358145 auch mehr als zwei Magnete, zum Beispiel vier Magnete, am Rotor vorgesehen. Das erhöht die Verstellkraft am Rotor beträchtlich.

Schließlich ist auch von Vorteil, wenn Toträume im Ventil beseitigt werden.Toträume sind Hohlräume, in denen der Liquor im Vergleich zu anderen Hohlräumen mit geringer Geschwindigkeit fließt oder sogar steht. Dort bilden sich leicht Gerinsel.

Die gefährlichen Hohlräume werden im Bereich des Bodens oder Deckels durch Ausfüllung beseitigt. Vorzugsweise wird der Hohlraum so weit ausgefüllt, daß der Durchflußquerschnitt zwischen dem Liquoreintritt und dem Liquoraustritt im Mittel nicht größer ist als 10 Quadratmillimeter, noch weiter bevorzugt im Mittel nicht größer ist als 7 Quadratmillimeter, und höchst bevorzugt im Mittel nicht größer ist als 4 Quadratmillimeter ist. Noch besser ist, wenn der Durchflußquerschnitt an keiner Stelle größer als 8 Quadratmillimeter ist, vorzugsweise an keiner Stelle größer als 5 Quadratmillimeter ist und noch weiter bevorzugt an keiner Stelle größer als 3Quadratmillimeter ist.

Zugleich soll der Durchflußquerschnitt zwischen dem Liquoreintritt und dem Ventilspalt zwischen Kugel und Kugelsitz bzw. zwischen dem Ventilspalt und dem Liquoraustritt an keiner Stelle kleiner als der Durchflußquerschnitt in dem Liquoreintritt sein. Der Ventilspalt zwischen Kugel und Kugelsitz kann sich dagegen bis auf Null reduzieren, wenn kein überschüssiges Liquor anfällt. Mit der erfindungsgemäßen Gestaltung des Durchflußquerschnittes soll die Strömungsgeschwindigkeit des Liquors im Ventil erhöht werden.

Günstig für die gewünschte Gestaltung des Durchflußquerschnittes kann ein scheibenariges Schwenkteil/Rotor sein, wenn die Liquorleitung entlang der Umfangsfläche des Schwenkteils/Rotors verläuft. Dabei können in der Umfangsfläche des Schwenkteils/Rotors und/oder in der korrespondierenden Ventil-Gehäusewand kanalbildende Rillen/Vertiefungen vorgesehen sein. Der Spalt zwischen Schwenkteil/Rotor und Ventil-Gehäusewand kann in herkömmlicher Weise abgedichtet werden. Vorzugsweise ist der Spalt so eng gestaltet, daß der Liquor nicht an dem Spalt austreten kann.

In der Zeichnung ist ein Ausführungsbeispiel der Erfindung dargestellt.

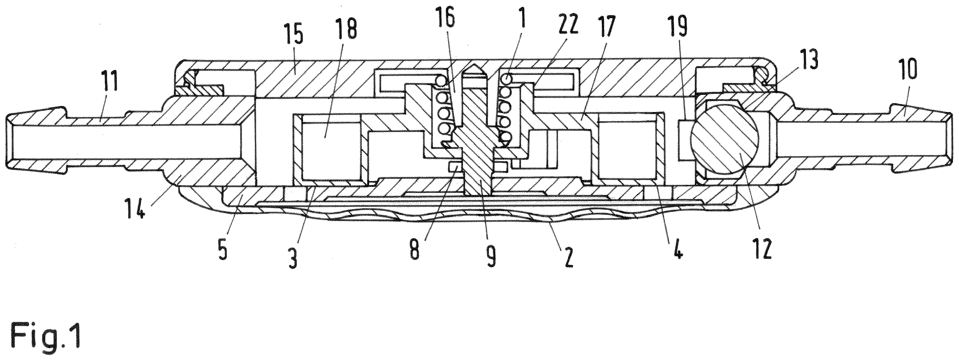

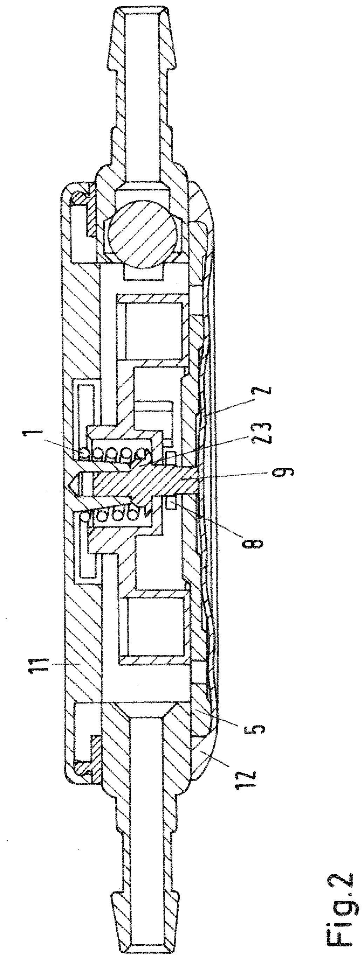

Fig. 1 und 2 zeigen gleichartige Schnitte durch ein erfindungsgemäßes, einstellbares Ventil. Dabei ist das Ventil in Fig. 1 verriegelt,arretiert und in Fig. 2 für eine Ventilverstellung entriegelt.

Das Ventil befindet sich in einer Drainleitung für Liquor. Die Eintrittsseite des Ventils ist mit 10 bezeichnet, die Austrittsseite mit 11.

Zu dem Ventil gehört ein Gehäuse mit einem Durchmesser von 17mm, das aus verschiedenen Teilen zusammen gesetzt ist. Mit 14 ist ein Gehäusering bezeichnet, mit 15 ein Boden, welcher den Gehäusering an einer Seite verschließt. Alle Gehäuseteile bestehen aus Titan(Titanlegierungen) und sind miteinander verschweißt. In anderen Ausführungsbeispielen werden alle Gehäuseteile miteinander verpreßt, so daß das Gehäuse dicht verschlossen ist.

Die dem Boden 15 des Gehäuses gegenüber liegende Gehäuseseite wird durch eine zweischalige Deckelkonstruktion verschlossen. Die innere Schale 5 gibt dieser Gehäuseseite die notwendige Stabilität und hat noch weitere, unten erläuterte Funktionen. Die äußere Schale der Deckelkonstruktion bildet eine von Hand verformbare Membran/Federblatt 2. Die äußere Schale/Membran/Federblatt 2 besitzt eine gewellte Form und soll sich unter äußerem Druck nach Erreichen eines labilen mittigen Zustandes schlagartig gegen die innere Schale/Widerlager 5 auswölben. Die innere Schale/Widerlager 5 ist dazu der Auswölbung angepaßt, welche die äußere Schale/Membran/Federblatt 2 einnimmt. Zur Anpassung ist eine gestufte Vertiefung zur Ventilmitte hin vorgesehen. Die innere Schale 5 bildet ein Widerlager für die äußere Schale/Membran/Federblatt 2. Als Widerlager begrenzt die innere Schale 5 die Bewegung der äußeren Schale/Membran/Federblatt 2, wenn ein behandelnder Arzt mit einer nicht dargestellten Verstelleinrichtung außen auf der Haupt des Patienten gegen die äußer Schale/Membran/Federblatt 2 drückt.

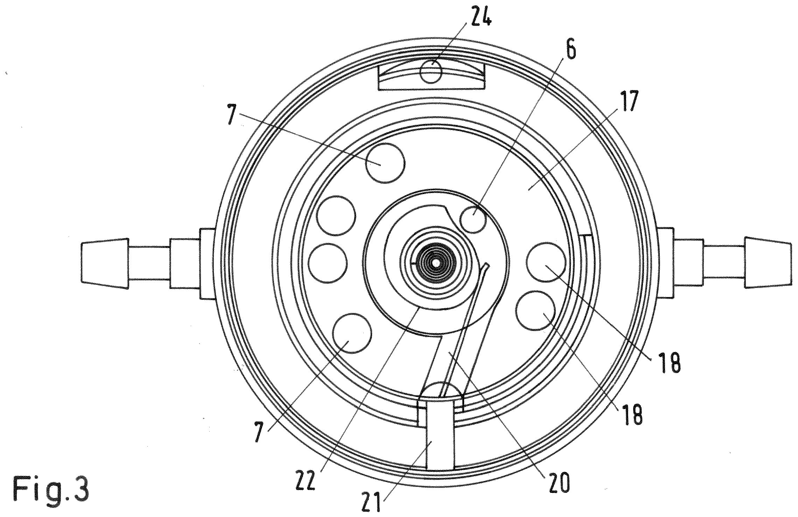

Eintrittsseitig ist im Gehäuse eine Ventilkugel 12 vorgesehen, die in einem Ventilsitz 13 liegt. Der Schließdruck der Ventilkugel wird je nach Position des Ventils durch deren Gewicht und durch eine an der Kugel 12 anliegenden Feder oder nur durch die Feder bestimmt. Von der Feder ist in Fig. 1 und 2 nur ein Ende 19 dargestellt. Dabei handelt es sich um eine Blattfeder, deren Ende 19 gemäß Fig. 3 in einem Bogen zu einer schwenkbeweglichen Halterung 21 für die Feder geführt ist. Der Bogen ist dem Krümmungsradius des Gehäuseringes angepaßt. Das andere Ende der Feder ist mit 20 bezeichnet und erstreckt sich von der Halterung 21 zu einer Kurvenbahn 22 eines im Gehäuse schwenkbeweglich angeordneten Schwenkteiles/Rotors 17. Wenn das Schwenkteil/Rotor 17 nach Fig. 2 von der inneren Schale/Widerlager 5 abgehoben hat, kann das Schwenkteil/Rotor 17mit der erwähnten Verstelleinrichtung verschwenkt werden. Dadurch erfährt das Federende 20 eine Biegung bzw. ein Drehmoment, das sich infolge der schwenkbeweglichen Halterung 21 auf das andere Federende 19 überträgt und zu einer Änderung des Schließdruckes an der Ventilkugel 12 führt.

Der Schwenkteil/Rotor 17 wird in der Verriegelungsstellung des Ventils nach Fig. 1 gegen die innere Schale/Widerlager 5 gedrückt. Dabei erlangt die Schale/Widerlager 5 mit ihrer Fläche 3 Reibungsschluß mit der Fläche 4 des Verstellteils/Rotors 17.

Der Verriegelungsdruck wird durch eine Spiralfeder 1 erzeugt, die sich an einem Ende an dem Boden 15 und am anderen Ende an einem Bund 23 einer Achse 9 abstützt. Die Achse 9 ist mit einem Ende axial verschiebbar und drehbeweglich in einer Führung 16 des Ventilbodens 15 geführt. Mit dem anderen Ende ist die Achse 9 verschiebbar und drehbeweglich in einer Lagerbohrung der inneren Schale/Widerlager 5 geführt.

Auf der Achse 9 sitzt der Schwenkteil/Rotor 17, wobei die Achse 9 in dem Schwenkteil/Rotor17 in axialer Richtung verschiebbar ist. In der Betriebsstellung "Verriegelung" nach Fig. 1 wird der Rotor 17 durch den Reibungsschluß mit dem Gehäuse an einer Drehung gehindert. Das dient der Sicherung der jeweiligen Ventilstellung.

In der Verriegelungsstellung steht die Achse 9 gegenüber der inneren Schale/Widerlager 5 vor.

Zum Lösen der Verriegelung wird die äußere Schale/Membran/Federblatt 2 nach innen gedrückt, bis die Schale/Membran/Federblatt 2 und die Achse 9 die in Fig. 2 dargestellte Stellung eingenommen haben. In der Stellung hat der Schwenkteil/Rotor 17 von der inneren Schale/Widerlager 5 abgehoben.

Auf dem Wege von der Stellung nach Fig. 1 zur Stellung nach Fig. 2 wölbt sich die äußere Schale/Membran/Federblatt 2 schlagartig gegen die innere SchaleWiderlage 5 aus. Das führt zu einem akustischen Signal; im Ausführungsbeispiel zu einem Knacken. Außerdem ist die Bewegung fühlbar. Das Knacken und die fühlbare Nachgiebigkeit zeigt dem behandelnden Arzt an, daß die Verriegelung aufgehoben worden ist.

Beim Entriegeln stellt ein mit der Achse 9 verpreßter Ring 8 sicher, daß keine übermäßige axiale Verschiebung der Achse 9 stattfindet. Außerdem kann die äußere Schale/Membran/Federblatt 2 die Achse 9 nicht weiter als bis zu inneren Schale/Widerlager 5 bewegen. Dort schließt das obere Ende der Achse 9 mit der inneren Schale/Widerlager ab.

Nach dem Entriegeln kann der Arzt mit der oben erwähnten Verstelleinrichtung den Schwenkteil/Rotor 17 auf der Achse 9 drehen, bis eine gewünschte Schwenkstellung und damit ein gewünschter neuer Schließdruck des Ventiles erreicht worden ist. Dann entlastet der Arzt die äußere Schale/Membran/Federblatt 2. Der Druck wird von der Membran 2 genommen, so daß sich die Membran wieder in die in Fig. 1 gezeigte Form zurückbilden kann. Zugleich wird die Achse 9 unter dem Druck der Feder 1 wieder in die Verriegelungsstellung gebracht und der zum Verriegeln notwendige Reibungsschluß erzeugt. Die Rückbildung der äußeren Schale/Membran/Federblatt führt zu einem weiteren Knackgeräusch, das der behandelnde Arzt als Verriegelungssignal versteht.

Zur Erzeugung des Entriegelungsdruckes drückt der behandelnde Arzt mit der nicht dargestellten bekannten, außen am Körper des Patienten angeordneten Verstelleinrichtung mittig gegen die Membran 2. Solche Verstelleinrichtungen sind in der DE10200803094.2 dargestellt und beschrieben.

Die Verstelleinrichtung ist mit Magneten versehen, die mit Magneten 18 zusammen wirken, welche in den Schwenkteil/Rotor 17 eingelassen sind. Nach der Entriegelung wird die Verstelleinrichtung von dem behandelnden Arzt gedreht.

Die Drehung der Verstelleinrichtung überträgt sich über die Magneten auf den Schenkteil/Rotor 17. Im Ausführungsbeispiel sind vier Magnete 18 in dem Schwenkteil/Rotor 17 angeordnet.

Im Ausführungsbeispiel ist die Schwenkbeweglichkeit des Schenkteiles/Rotors 17durch Anschläge 6 beschränkt. Die Anschläge 6 sind Stifte. In anderen Ausführungsbeispielen können anstelle der Stifte beliebige andere Bauteile als Anschläge eingesetzt werden.

Im Ausführungsbeispiel ist der Schwenkteil/Rotor 17 im Übrigen mit Markierungen versehen, welche dem behandelnden Arzt erlauben, die jeweilige Schwenkteil/Rotorstellung beim Röntgen zu kontrollieren. Bei den Markierungen handelt es sich um Bohrungen 7.

Schließlich sind am Gehäuse noch Markierungen 24 angebracht, die beim Röntgen die Kontrolle erlauben, ob das Ventil richtig implantiert worden ist.

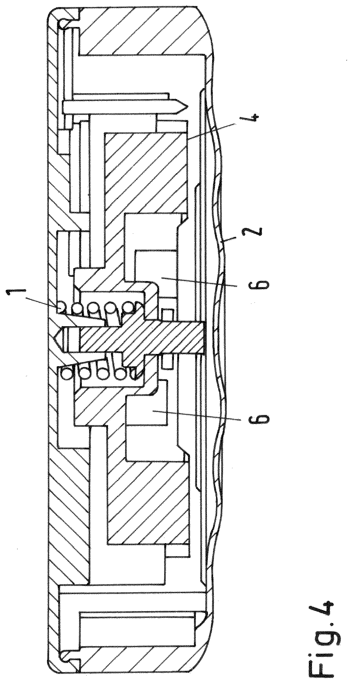

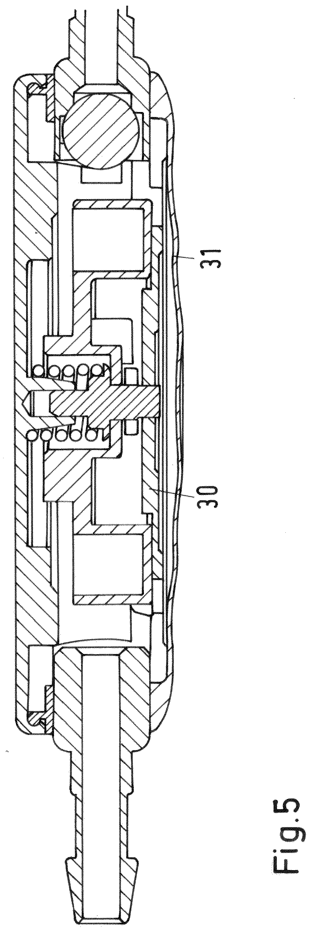

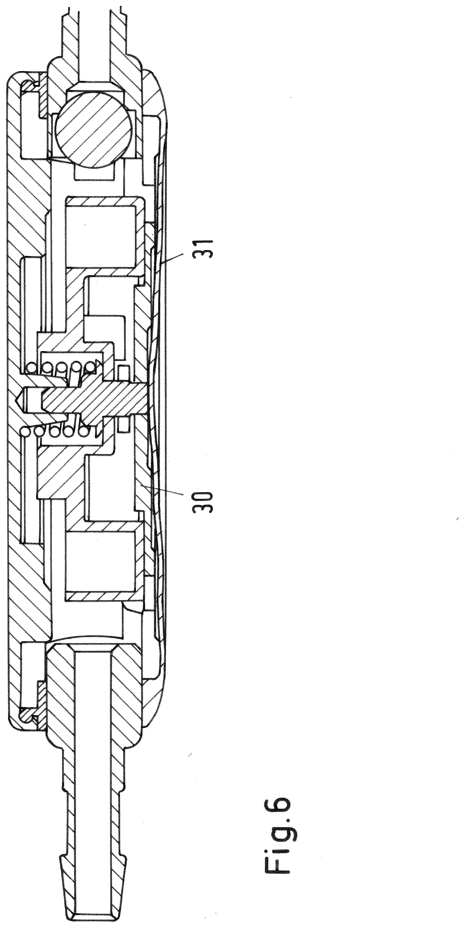

Fig. 5 und 6 zeigen ein weiteres Hydrocephalusventil mit zweischaligem Ventildeckel. In allen anderen Bestandteilen stimmt das Ventil nach Fig. 5 und 6 mit dem Ventil nach Fig. 1 bis 4 überein.

Von dem zweischaligen Ventildeckel entspricht die innere Schale/Widerlager 30 der inneren Schale/Widerlager 5 nach Fig. 1 bis 4. Ein Unterschied ergibt sich bei der äußeren Schale/Membran/Federblatt 31.

In der äußeren Schale/Membran/Federblatt 31ist eine Beulstruktur vorgesehen, die in der Ausgangsstellung nach Fig.5 durch eine gestufte Ausbeulung gekennzeichnet ist. In jeder Stufe sind eben und parallel zum Boden des Ventilgehäuses verlaufende ausgebeulte Flächen vorgesehen. Zwischen den verschiedenen Stufen sind geneigt verlaufende Übergangsflächen vorgesehen. Die dargestellten Beulen lassen sich leichter in das Ausgangsmaterial einarbeiten als die Wellenstruktur nach Fig. 1 bis 4.

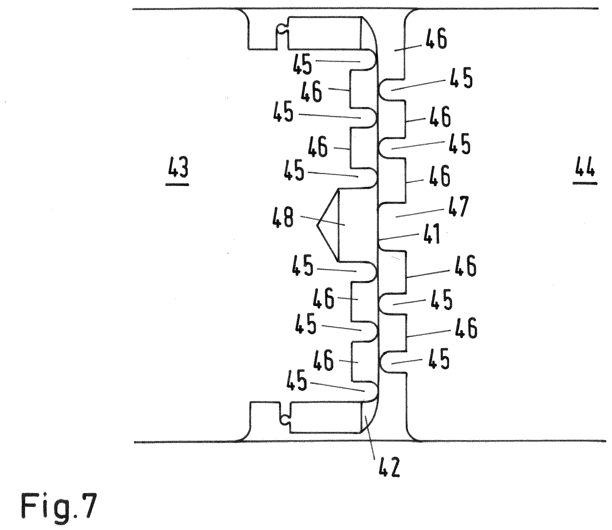

Fig. 7 zeigt eine Preßvorrichtung zur Erzeugung einer Wellenform in einer Schale für einen zweischaligen Gehäusedeckel aus einer Titanlegierung. Dabei besteht die Schale im Beispiel aus einer kreisförmigen Folie 41 mit einer Dicke von 0,15 mm und einem Ring 42. Die Folie 41 ist mit dem Ring 42 im Ausführungsbeispiel als Drehteil auf einer Drehbank einstückig hergestellt. In anderen Ausführungsbeispielen sind die Folie 41 und der Ring 42 als separate Teile herstellt und miteinander verschweißt worden. Durch die Verbindung ist eine topfförmige Schale für einen zweischaligen Gehäusedeckel mit ebener Oberfläche entstanden. Der Durchmesser der Folie beträgt 14,6mm. Nach der Ausbeulung wird die Schale als äußere Schale mit einer zweiten Schale als innere Schale zu einem Deckel verbunden. Der Deckel ist für ein Hydrocephalusventil mit einem Durchmesser von 17mm bestimmt.

In der dargestellten Ausgangsform wird die äußere Schale vor der Verbindung mit der inneren Schale mit einer Beulstruktur versehen. Dazu liegt die Schale in einer Presse mit einer Matrize 43 und einer Patrize 44. Die Patrize und die Matrize besitzen Auswölbungen 45 und Vertiefungen 46. Dabei liegt jeweils eine Auswölbung 45 gegenüber einer Vertiefung 46. Außerdem ist eine mittige Auswölbung 47 vorgesehen, die einer mittigen Vertiefung 48 gegenüberliegt. Die Fig. 7 beinhaltet eine Schnittdarstellung, so daß jeweils zwei Auswölbungen 45 mit gleichem Abstand zur Mitte der Schale zu einem Ring an der Matrize und an der Patrize gehören.

Dementsprechend gehören auch jeweils zwei Vertiefungen mit gleichem Abstand zur Mitte der Schale zu einer ringförmigen Vertiefung.

In einem ersten Verformungsschritt werden die Matrize und die Patrize gegeneinander bewegt, so daß die Auswölbungen 45 gegen die Folie 41 drücken und Wellen von 0,5mm Höhe verursacht werden.

Die Form der sich bildenden Wellen ist von der Rundung der Auswölbungen 45 an deren Berührungsfläche mit der Folie 41 abhängig. Bei großen Rundungen können zum Beispiel sinusartig verlaufende Wellen erzeugt werden. Bei kleinen(scharfen) Rundungen können annähernd zick-zackförmig verlaufende Wellen entstehen.

Nach einer Entlastung der Folie durch Zurückfahren von Matrize und Patrize verbleiben im Ausführungsbeispiel Wellen mit einer Höhe von etwa 0,25 mm. Danach ist die Folie 41 viel beweglicher geworden. Unter einer Belastung von 1 kg wölbt sich die verformte Folie 41 um 0,2mm aus, während sich eine unverformte, ebene Folie 41 nur um 0,1mm auswölbt.

In einem zweiten Verformungsschritt wird die Schale um weitere 1mm ausgewölbt. Im unbelasteten Zustand ergibt sich dann eine Auswölbung der Folie 41 von 0,3mm.

Die so entstandene Schale (aus Ring 42 und Folie 41) kann mit einer mittig angreifenden Kraft von 6 bis 7N um 0,6mm eingedrückt werden. Dabei entsteht ein Knackgeräusch. Die entstandene Schale formt kehrt nach einer Eindrückung selbsttätig in die Ausgangsform zurück, wenn die Schale wieder entlastet wird. In der Anwendung auf zweischalige Ventildeckelkonstuktionen nach Fig. 1 bis 6 ist eine selbsttätige Rückkehr der äußeren Schale nach einer Einbeulung in die Ausgangsform nicht zwingend erforderlich, weil bei einer Einbeulung der äußerenen Schale gegen eine Federkraft gearbeitet werden muß, welche nach Entlastung der äußeren Schale vom Druck der Verstelleinrichtung die zusammen gedrückte Feder die Rückstellung/Rückformung der äußeren Schale in die Ausgangsform initiiert. Die selbsttätige Rückstellung bildet aber eine vorteilhafte Sicherheit für die Rückstellung/Rückformung der äußeren Schale.

In Ausführungsbeispielen von äußeren Schalen für Ventildeckel mit anderem Durchmesser finden bei kleineren Durchmessern dünnere Folien Anwendung, bei größeren Durchmessern dickere Folien. Dabei wird die Foliendicke zum Beispiel in Schritten von wenigen Hundertstel Millimetern verändert, um unter sonst gleichen Umständen zu vergleichbaren Ergebnissen zu kommen.

## Patentansprüche

1. Einstellbares Hydrocephalus-Ventil zum Druckausgleich des Liquor im Schädel eines Hydrocephalus-Patienten, wobei das Ventil dem Patienten implantierbar ist und vorzugsweise mit einer gleichfalls implantierbaren Schlauchleitung, über die überschüssiger Liquor aus dem Schädel des Patienten abziehbar und in die obere Hohlvene oder in den Bauchraum drainierbar ist, wobei der Ventildruck durch eine Feder (20) bestimmt wird und wobei die Feder (20) über eine Verstellmechanik verstellt wird, so daß die Feder (20) gespannt oder entlastet wird,
wobei zu der Verstellmechanik des Ventils ein schwenkbewegliches oder drehbewegliches and im Ventil angeordnetes Schwenkteil/Rotor (17) gehört, das mit Magneten versehen ist und von außen durch Schwenken oder Drehen einer Verstelleinrichtung bewegbar ist, die ihrerseits mit Magneten versehen ist,
wobei das Ventil mit einer Verriegelung für den Schwenkteil/Rotor (17) versehen ist, so daß zwischen zwei Verstellvorgängen eine Arretierung des Schwenkteiles/Rotors (17) erfolgen kann, und wobei die Verriegelung durch Reibungsschluß und/oder durch eine Verzahnung zwischen dem Schwenkteil/Rotor (17) und dem Gehäuse erfolgt und wobei das drehbewegliche oder schwenkbewegliche Schwenkteil/Rotor (17) zur Aufhebung des Reibungsschlusses bzw. zur Aufhebung des Zahneingriffes unter Eindrückung des Ventildeckels (2) in axialer Richtung bewegbar ist,
wobei die Entriegelungsbewegung gegen eine Federkraft erfolgt, die bei der Verriegelung den Reibungsschluß bzw. den Zahneingriff bewirkt,
wobei das Ventil mit einer von einer Gehäuseverformung unabhängigen Feder (1) für die Herbeiführung des Reibungsschluß bzw. des Zahneingriffs versehen ist, wobei die Feder (1) zwischen dem Schwenkteil/Rotor (17) und dem Gehäuseboden (15) vorgesehen ist,
**dadurch gekennzeichnet, daß** der Ventildeckel (2) durch ein Federblatt gebildet ist, das vor der Entriegelung eine Beule aufweist, die durch Eindrücken mit der Verstelleinrichtung schlagartig in eine gegenüber liegende Beule bringbar ist.

2. Ventil nach Anspruch 1, **dadurch gekennzeichnet, daß** der Ventildeckel (2) mindestens zweischalig ausgebildet ist.

3. Ventil nach Anspruch 1 oder 2, **dadurch gekennzeichnet, daß** der Schwenkteil/Rotor (17) auf einer Achse angeordnet ist, die im Ventil in Bezug auf den verformbaren Ventildeckel (2) ein separates Bauteil bildet.

4. Ventil nach einem der Ansprüche 1 bis 3, **gekennzeichnet durch** akustische Signalgeber und/oder optische Signalgeber und/oder gefühlte Signale.

5. Ventil nach einem der Ansprüche 1 bis 4, **gekennzeichnet durch** ein mechanische und/oder elektronische Signalgeber.

6. Ventil nach einem der Ansprüche 1 bis 5, **gekennzeichnet durch** eine eindrückbare Ausbeulung des Ventildeckels (2), wobei durch Eindrückung eine stabile gegenüberliegende Beule erzeugbar ist und die gegenüberliegende Beule mit Gegendruck in die ursprüngliche Ausbeulung rückformbar ist.

7. Ventil nach Anspruch 6, **gekennzeichnet durch** eine mehrstufige Verformung des Ventildeckels (2).

8. Ventil nach Anspruch 6 oder 7, **dadurch gekennzeichnet, daß** der Ventildeckel (2) zweischalig ausgebildet ist, wobei die äußere Schale eindrückbar ist und die innere Schale ein Widerlager für die äußere Schale bildet.

9. Ventil nach Anspruch 8, **dadurch gekennzeichnet, daß** der Hub der äußeren Schale kleiner als der Hub für die Ausbildung einer stabilen gegenüberliegenden Beule ist.

10. Ventil nach Anspruch 9, **dadurch gekennzeichnet, daß** bei einem zweischaligen Ventildeckel (2) die innere Schale den Hub der äußeren Schale begrenzt.

11. Ventil nach einem der Ansprüche 1 bis 10, **dadurch gekennzeichnet, daß** das drehbewegliche oder schwenkbewegliche Schwenkteil/Rotor (17) mit einer Achse, Zapfen oder Bolzen drehbeweglich oder schwenkbeweglich gelagert ist und dass die Achse/Zapfen/Bolzen zugleich in axialer Richtung verschiebbar angeordnet ist, wobei ein Verschiebungsweg in die eine axiale Richtung eine Endstellung und ein Verschiebeweg in die entgegengesetzte axiale Richtung eine zweite Endstellung hat, wobei die eine Endstellung bei der Verschiebung die Entriegelungsstellung ist und die andere Endstellung die Verriegelungsstellung ist.

12. Ventil nach Anspruch 11, **dadurch gekennzeichnet, daß** für die Verschiebung in die Verriegelungsstellung als Antrieb eine Feder vorgesehen ist, die von dem Antrieb für die Verschiebung in die Entriegelungsstellung unabhängig ist.

13. Ventil nach einem der Ansprüche 1 bis 12, **dadurch gekennzeichnet, daß** als Antrieb für die Verschiebung in die Entriegelungsstellung die außen am Körper des Patienten angeordnete Verstelleinrichtung vorgesehen ist.

14. Ventil nach einem der Ansprüche 1 bis 13, **dadurch gekennzeichnet, daß** das ventildeckelseitige Ende der Achse, Zapfen oder Bolzen (9) in der Entriegelungsstellung mit der Oberfläche der inneren Schale des zweischaligen Ventildeckels (2) bündig abschließt und in der Verriegelungsstellung gegenüber der Oberfläche der inneren Schale nach außen vorsteht.

15. Ventil nach einem der Ansprüche 1 bis 14, **dadurch gekennzeichnet, daß** das innen im Ventil angeordnete schwenkbewegliche oder drehbewegliche Schwenkteil/Rotor (17) mindestens eine Bohrung zur Bestimmung der Lage des Schwenkteiles/Rotors (17) aufweist.

16. Ventil nach Anspruch 15, **gekennzeichnet durch** Anschläge in dem Ventil zur Begrenzung der Schwenkbewegung des Schwenkteiles/Rotors (17) vorzugsweise durch Zapfen als Anschläge.

17. Ventil nach einem der Ansprüche 1 bis 16, **gekennzeichnet durch** eine Markierung am Ventil zur Bestimmung der Implantierungslage.

## Claims

1. An adjustable hydrocephalus valve for equalising the pressure of the cerebrospinal fluid in the skull of a hydrocephalus patient, wherein the valve can be implanted in the patient and is preferably provided with a hose line which can also be implanted and via which excess cerebrospinal fluid can be withdrawn from the skull of the patient and drained into the superior vena cava or into the abdominal cavity, wherein the valve pressure is determined by a spring (20) and wherein the spring (20) is adjusted via an adjusting mechanism so that the spring (20) is tensioned or relieved,
wherein the adjustment mechanism of the valve includes a pivotable or rotatable pivot member/rotor (17) arranged in the valve, which is provided with magnets and is movable from the outside by pivoting or rotating an adjustment device, which in turn is provided with magnets,
wherein the valve is provided with a locking device for the pivot member/rotor (17), so that the pivot member/rotor (17) can be locked between two adjustment processes, and wherein the locking is effected by frictional engagement and/or by a toothing between the pivot member/rotor (17) and the housing, and wherein the rotatable or pivotable pivot member/rotor (17) can be moved in the axial direction for cancelling the frictional engagement or for cancelling the engagement of the teeth by pressing in the valve cover (2) in the axial direction,
wherein the unlocking movement takes place against a spring force which causes the frictional engagement or the tooth engagement during locking,
wherein the valve is provided with a spring (1), which is independent of a housing deformation, for producing the frictional engagement or the tooth engagement,
wherein the spring (1) is provided between the pivot member/rotor (17) and the housing base (15),
**characterised in that** the valve cover (2) is formed by a spring leaf which has a bump before unlocking which can be abruptly brought into an opposite bump by pressing it in with the adjusting device.

2. Valve according to claim 1, **characterised in that** the valve cover (2) is of at least two-shell design.

3. Valve according to claim 1 or 2, **characterised in that** the pivot member/rotor (17) is arranged on an axis which forms a separate component in the valve in relation to the deformable valve cover (2).

4. Valve according to any one of claims 1 to 3, **characterised by** acoustic signal transmitters and/or optical signal transmitters and/or sensed signals.

5. Valve according to one of claims 1 to 4, **characterised by** a mechanical and/or electronic signal transmitter.

6. A valve according to any one of claims 1 to 5, **characterised by** a depressible bump of the valve cover (2), wherein a stable opposite bump can be produced by depression and the opposite bump can be formed back into the original bump with counterpressure.

7. Valve according to claim 6, **characterised by** a multi-stage deformation of the valve cover (2).

8. Valve according to claim 6 or 7, **characterised in that** the valve cover (2) is of two-shell design, the outer shell being pressable in and the inner shell forming an abutment for the outer shell.

9. Valve according to claim 8, **characterized in that** the stroke of the outer shell is smaller than the stroke for the formation of a stable opposite bump.

10. Valve according to claim 9, **characterised in that**, in the case of a two-shell valve cover (2), the inner shell limits the stroke of the outer shell.

11. A valve according to any one of claims 1 to 10, **characterised in that** the rotatable or pivotable pivot member/rotor (17) is rotatably or pivotably mounted with an axle, pin or bolt, and **in that** the axle/pin/bolt is at the same time arranged to be displaceable in the axial direction, a displacement path in one axial direction having one end position and a displacement path in the opposite axial direction having a second end position, one end position being the unlocking position and the other end position being the locking position during the displacement.

12. Valve according to claim 11, **characterised in that** a spring is provided as drive for the displacement into the locking position, the spring being independent of the drive for the displacement into the unlocking position.

13. Valve according to one of the claims 1 to 12, **characterised in that** the adjusting device arranged on the outside of the patient's body is provided as the drive for the displacement into the unlocking position.

14. Valve according to one of the claims 1 to 13, **characterised in that** the valve cover-side end of the axle, pin or bolt (9) is flush with the surface of the inner shell of the two-shell valve cover (2) in the unlocked position and projects outwards relative to the surface of the inner shell in the locked position.

15. Valve according to any one of claims 1 to 14, **characterised in that** the pivotable or rotatable pivot member/rotor (17) arranged inside the valve has at least one bore for determining the position of the pivot member/rotor (17).

16. Valve according to claim 15, **characterised by** stops in the valve for limiting the pivoting movement of the pivot member/rotor (17), preferably by pins as stops.

17. Valve according to any one of claims 1 to 16, **characterised by** a marking on the valve for determining the implantation position.

## Revendications

1. Soupape d'hydrocéphalie réglable pour équilibrer la pression du liquide céphalorachidien dans le crâne d'un patient atteint d'hydrocéphalie, la soupape pouvant être implantée chez le patient et de préférence avec un tuyau flexible également implantable, par lequel le liquide céphalorachidien en excès peut être retiré du crâne du patient et drainé dans la veine cave supérieure ou dans la cavité abdominale, la pression de la soupape étant déterminée par un ressort (20) et le ressort (20) étant réglé par un mécanisme de réglage, de sorte que le ressort (20) est tendu ou déchargé, dans lequel le mécanisme de réglage de la soupape comprend une partie pivotante/un rotor (17) mobile en rotation ou en pivotement disposé dans la soupape, la partie pivotant/le rotor étant pourvu d'aimants et qui peut être déplacé de l'extérieur par pivotement ou rotation d'un dispositif de réglage qui est lui-même pourvu d'aimants,
la soupape étant pourvue d'un verrouillage pour la partie pivotante/le rotor (17), de sorte qu'un blocage de la partie pivotante/du rotor (17) peut avoir lieu entre deux opérations de réglage, et le verrouillage s'effectuant par une liaison par friction et/ou par une denture entre la partie pivotante/le rotor (17) et le boîtier, et la partie pivotante/le rotor (17) mobile en rotation ou en pivotement peut être déplacé(e) dans la direction axiale pour supprimer la liaison par friction ou pour supprimer la liaison par la denture en enfonçant le couvercle de soupape (2) ,
le mouvement de déverrouillage s'effectuant à l'encontre d'une force élastique qui, lors du verrouillage, provoque la liaison par friction ou la liaison par la denture,
la soupape étant pourvue d'un ressort (1) indépendant d'une déformation du boîtier pour provoquer la liaison par friction ou la liaison par la denture,
le ressort (1) étant prévu entre la partie pivotante/le rotor (17) et le fond du boîtier (15)
**caractérisé en ce que** le couvercle de soupape (2) est formé par une lame de ressort qui présente une bosse avant le déverrouillage, laquelle peut être amenée brusquement dans une bosse opposée par enfoncement avec le dispositif de réglage .

2. Soupape selon la revendication 1, **caractérisée en ce que** le couvercle de soupape (2) est réalisé avec au moins deux coquilles.

3. Soupape selon la revendication 1 ou 2, **caractérisée en ce que** la partie pivotante/le rotor (17) est disposé(e) sur un axe qui constitue un composant séparé dans la soupape par rapport au couvercle de soupape déformable (2).

4. Soupape selon l'une des revendications 1 à 3, **caractérisée par** des générateurs de signaux acoustiques et/ou des générateurs de signaux optiques et/ou des signaux ressentis.

5. Soupape selon l'une des revendications 1 à 4, **caractérisée par** un générateur de signaux mécaniques et/ou électroniques.

6. Soupape selon l'une des revendications 1 à 5, **caractérisée par** un renflement enfonçable du couvercle de soupape (2), un renflement opposé stable pouvant être généré par enfoncement et le renflement opposé pouvant être reformé avec une contre-pression dans le renflement initial.

7. Soupape selon la revendication 6, **caractérisée par** une déformation en plusieurs étapes du couvercle de soupape (2).

8. Soupape selon la revendication 6 ou 7, **caractérisée en ce que** le couvercle de soupape (2) est réalisé en deux coquilles, la coquille extérieure pouvant être enfoncée et la coquille intérieure formant un appui pour la coquille extérieure.

9. Soupape selon la revendication 8, **caractérisée en ce que** la course de la coquille extérieure est inférieure à la course nécessaire pour former une bosse opposée stable.

10. Soupape selon la revendication 9, **caractérisée en ce que**, dans le cas d'un couvercle de soupape (2) à deux coquilles, la coquille intérieure limite la course de la coquille extérieure.

11. Soupape selon l'une des revendications 1 à 10, **caractérisée en ce que** la pièce pivotante/le rotor (17) mobile en rotation ou en pivotement est monté(e) mobile en rotation ou en pivotement avec un axe, un tourillon ou un boulon et **en ce que** l'axe/le tourillon/le boulon est disposé(e) de manière à pouvoir être déplacé(e) en même temps dans la direction axiale, une course de déplacement dans une direction axiale ayant une première position finale et une course de déplacement dans la direction axiale opposée ayant une deuxième position finale, l'une des positions finales lors du déplacement étant la position de déverrouillage et l'autre position finale étant la position de verrouillage.

12. Soupape selon la revendication 11, **caractérisée en ce que** pour le déplacement vers la position de verrouillage, il est prévu comme entraînement un ressort qui est indépendant de l'entraînement pour le déplacement vers la position de déverrouillage.

13. Soupape selon l'une des revendications 1 à 12, **caractérisée en ce que** le dispositif de réglage disposé à l'extérieur du corps du patient est prévu comme entraînement pour le déplacement vers la position de déverrouillage.

14. Soupape selon l'une des revendications 1 à 13, **caractérisée en ce que** l'extrémité côté couvercle de soupape de l'axe, du tourillon ou du boulon (9) affleure la surface de la coquille intérieure du couvercle de soupape à deux coquilles (2) dans la position de déverrouillage et fait saillie vers l'extérieur par rapport à la surface de la coquille intérieure dans la position de verrouillage.

15. Soupape selon l'une des revendications 1 à 14, **caractérisée en ce que** la pièce pivotante/le rotor (17) mobile en rotation ou en pivotement disposé(e) à l'intérieur de la soupape présente au moins un alésage pour déterminer la position de la pièce pivotante/du rotor (17).

16. Soupape selon la revendication 15, **caractérisée par** des butées dans la soupape pour limiter le mouvement de pivotement de la partie pivotante/du rotor (17), de préférence par des tourillons servant de butées.

17. Soupape selon l'une des revendications 1 à 16, **caractérisée par** un marquage sur la soupape pour déterminer la position de l'implantation.
